**Europäisches Patentamt**

**European Patent Office**

**Office européen des brevets**

(19)

(11) Veröffentlichungsnummer: **0 218 144**
**B1**

# EUROPÄISCHE PATENTSCHRIFT

(12)

(45) Veröffentlichungstag der Patentschrift:
25.04.90

(51) Int. Cl.⁴: **A61M 5/315, B05C 17/00**

(21) Anmeldenummer: **86113143.1**

(22) Anmeldetag: **24.09.86**

(54) **Injektions-Spritzpistole mit einstellbarer Druckbegrenzung.**

(30) Priorität: **25.09.85 DE 3534215**

(43) Veröffentlichungstag der Anmeldung:
**15.04.87 Patentblatt 87/16**

(45) Bekanntmachung des Hinweises auf die Patenterteilung:
**25.04.90 Patentblatt 90/17**

(84) Benannte Vertragsstaaten:
**AT BE CH DE FR GB IT LI LU NL SE**

(56) Entgegenhaltungen:
**US-A- 2 735 431**
**US-A- 3 110 310**

(73) Patentinhaber: **Henke-Sass, Wolf GmbH,**
**Kronenstrasse 16, D-7200 Tuttlingen(DE)**

(72) Erfinder: **Jonischkeit, Horst, Schumannstrasse 40,**
**D-7200 Tuttlingen(DE)**

(74) Vertreter: **Fehners, Klaus Friedrich, Dipl.-Ing.**
**Dipl.-Wirtsch.-Ing. et al, Patentanwälte Dipl.-Ing. A.**
**Wedde Dipl.-Ing. K. Empl Dipl.-Ing., Dipl.-Wirtsch.-Ing. K.**
**Fehners Schumannstrasse 2, D-8000 München 80(DE)**

## Beschreibung

Die Erfindung betrifft eine Injektions-Spritzpistole mit einer, für den Ansatz einer für die Aufnahme einer Zylinderampulle vorgesehenen Hülse ausgebildeten Halterung, einem mit dieser verbundenen Handgriff und einem daran schwenkbar befestigten Bedienungshebel und einer, in der Halterung geführten und mit dem Bedienungshebel verbundenen, die Verschiebung eines in der Zylinderampulle befindlichen Kolbens bewirkende Druckstange.

Solche Injektions-Spritzpistolen werden in vielfältigen medizinischen Bereichen angewandt, insbesondere auch von Zahnärzten für die intraligamentale Anästhesie. Vorteil solcher Spritzpistolen ist, dass mit ihnen ein verhältnismässig hoher Druck für einzelne Spritzvorgänge erzeugt werden kann.

Nachteilig ist aber andererseits bei solchen Injektions-Spritzpistolen, dass der das Instrument bedienende Arzt den erzeugten Druck rein gefühlsmässig kontrollieren bzw. begrenzen muss, um beispielsweise zu verhindern, dass nicht etwa einerseits die eingesetzte Zylinderampulle mit der zu spritzenden Lösung birst oder andererseits ein zu behandelnder Zahn aus seinem paradontösen Zahnbett herausgelöst bzw. zumindest gelockert wird.

Aufgabe der Erfindung ist es deshalb, die bekannte Injektions-Spritzpistole so auszubilden, daß der von der Hebelmechanik auf die zu spritzende Flüssigkeit ausgeübte Druck begrenzt und sogar eingestellt und reguliert werden kann.

Diese Aufgabe wird dadurch gelöst, daß bei einer Injektions-Spritzpistole der eingangs genannten Art eine, auf Bedienungshebel und Druckstange einwirkende Druckbegrenzungsvorrichtung mit einem Federelement und einem zweiarmigen Umlenkhebel vorgesehen ist, wobei der Bedienungshebel unter Zwischenschaltung des zweiarmigen Umlenkhebels am Handgriff befestigt, der zweiarmige Umlenkhebel auf einer im Handgriff angeordneten Achse schwenkbar gelagert und der Bedienungshebel mit seiner Drehachse an dem ersten Arm des Umlenkhebels schwenkbar befestigt ist und das Federelement an dem zweiten Arm des Umlenkhebels angreift.

Hierbei kommt dem Umlenkhebel die Funktion eines Widerlagers für den Bedienungshebel zu, wobei diese Widerlager dem durch den Bedienungshebel verursachten Druck bei Erreichung einer bestimmten Größe ausweichen und bei Nachlassen dieses Druckes in die Ausgangsstellung zurückkehren kann. Das Federelement bildet die einstellbare Widerlagerkraft.

Vorteilhaft ist das Federelement als Spiralfeder ausgebildet und auf einer Führungsstange angeordnet, wobei diese Führungsstange mit ihrem ersten Ende am zweiten Arm des Umlenkhebels gelenkig gelagert ist und mit ihrem zweiten Ende in eine, im Handgriff eingesetzte Stellschraube hineinragt und das untere Ende des Federelementes auf dem Rand der Stellschraube anliegt.

Diese vorbeschriebene Kombination aus Federelement und Führungsstange erlaubt die genaue Einstellbarkeit des von dem Federelement bewirkten Widerlagerdruckes.

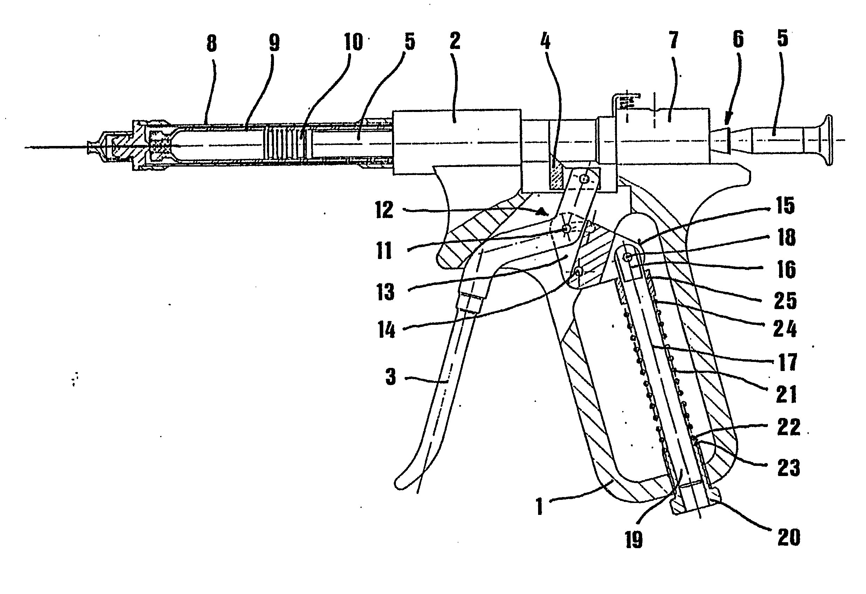

Ein Ausführungsbeispiel der Erfindung wird nachfolgend anhand der beigefügten Zeichnung näher erläutert. Diese zeigt eine teilweise aufgeschnittene Seitenansicht einer erfindungsgemässen Injektions-Spritzpistole.

Die in der Zeichnung veranschaulichte Injektions-Spritzpistole besteht im wesentlichen aus einem Handgriff 1, einer mit diesem einstückig ausgebildeten Halterung 2 und einem, im Handgriff schwenkbar gelagerten Bedienungshebel 3, welcher über eine im Handgriff eingebaute und nachfolgend näher erläuterte Hebelmechanik eine Klinke 4 betätigt, welche wiederum in an der Druckstange 5 ausgebildete Rasten 6 eingreift und den Vorschub der Druckstange 5 nach jeweiliger Betätigung des Bedienungshebels 3 bewirkt.

Die Druckstange 5 ist in der Halterung 2 sowie in einem zusätzlichen Führungsstück 7 am Handgriff 1 geführt.

In die Halterung 2 ist eine Hülse 8 eingesetzt, in welcher widerum eine, das einzuspritzende Medikament enthaltende Zylinderampulle 9 mit einem diese nach aussen abschliessenden Kolben 10 eingelegt ist. Das hülsenseitige Ende der Druckstange 5 kommt zur Anlage an dem Kolben 10 und drückt den Kolben während des Spritzvorganges in die Zylinderampulle hinein.

Der Bedienungshebel 3 ist mit seiner Drehachse 11 nicht unmittelbar im Handgriff 1, sondern an dem ersten Arm 12 eines zweiarmigen Umlenkhebels 13 drehbar gelagert, welcher wiederum im Handgriff 1 drehbar um eine Achse 14 befestigt ist. Am zweiten Arm 15 des zweiarmigen Umlenkhebels 13 ist das obere Ende 16 einer Führungsstange 17 drehbar über die Achse 18 befestigt, wobei das untere Ende 19 der Führungsstange 17 in eine, als Hohlkörper ausgebildete Stellschraube 20 hineinragt, welche wiederum in den Handgriff 1 längenverstellbar eingesetzt ist.

Auf der Führungsstange 17 ist ein, als Spiralfeder ausgebildetes Federelement 21 angeordnet, welches mit seinem unteren Ende 22 gegen den Rand 23 der Stellschraube 20 zur Anlage kommt und mit seinem oberen Ende 24 an einer, auf der Führungsstange 17 angeordneten Hülse 25 anliegt.

Die Funktionsweise der vorbeschriebenen Hebelmechanik lässt sich wie folgt darstellen:

Der die Injektionspistole bei einer Injektion betätigende Arzt bewegt den Bedienungshebel 3 gegen den Handgriff 1, wobei sich der Bedienungshebel 3 um die Drehachse 11 dreht und die Klinke 4 zusammen mit der Druckstange 5 gegen den Kolben 10 bewegt.

Die Drehachse bzw. der Drehpunkt 11 des Bedienungshebels 3 auf dem Umlenkhebel 13 bildet, stabilisiert durch die Kraft des Federelementes 21, das Widerlager, welches jedoch bei einer, die Kraft des Federelementes 21 übersteigende Kraft aus dem Bedienungshebel 3 ausweicht, wobei sich der Umlenkhebel 13 um seine Befestigungsachse 14 im Handgriff 1 dreht und die Führungsstange 17 in die als Hohlkörper ausgebildete Stellschraube 20 hineintaucht, wobei durch dieses Ausweichen des Lagerpunktes 11 der Bedienungshebel 3 bis an den mechanischen Anschlag der Führungsstange 17 in der Stellschraube 20 heruntergedrückt werden kann, ohne dass sich der Injektionsdruck erhöht.

Bei ganz durchgedrücktem Bedienunghebel 3 und ausgelenktem Umlenkhebel 13 übernimmt das Federelement 21 die Injektion bis der Lagerpunkt 11 wieder in die Ausgangslage zurückgedrückt wurde.

Diese druckregulierte Hebelmechanik gewährleistet einerseits, dass ein Bersten der Zylinderampulle wegen zu hohen Druckes nicht mehr möglich ist, andererseits auch insbesondere paradontöse Zähne bei intraligamentaler Injektion nicht etwa aus ihrem Zahnbett herausgelöst werden können.

**Patentansprüche**

1. Injektions-Spritzpistole mit einer, für den Ansatz einer für die Aufnahme einer Zylinderampulle (9) vorgesehenen Hülse (8) ausgebildeten Halterung (2), einem mit dieser verbundenen Handgriff (1) und einem daran schwenkbar befestigten Bedienungshebel (3) und einer in der Halterung (2) geführten und mit dem Bedienungshebel (3) verbundenen, die Verschiebung eines in der Zylinderampulle (9) befindlichen Kolbens (10) bewirkenden Druckstange (5), dadurch gekennzeichnet, daß eine auf den Bedienungshebel (3) und die Druckstange (5) einwirkende Druckbegrenzungsvorrichtung mit einem Federelement (21) und einem zweiarmigen Umlenkhebel (13) vorgesehen ist, wobei der Bedienungshebel (3) unter Zwischenschaltung des zweiarmigen Umlenkhebels (13) am Handgriff (1) befestigt, der zweiarmige Umlenkhebel (13) auf einer im Handgriff (1) angeordneten Achse (14) schwenkbar gelagert und der Bedienungshebel (3) mit seiner Drehachse (11) an dem ersten Arm (12) des Umlenkhebels (13) schwenkbar befestigt ist und das Federelement (21) an dem zweiten Arm (15) des Umlenkhebels (13) angreift.

2. Injektions-Spritzpistole nach Anspruch 1, dadurch gekennzeichnet, daß das Federelement (21) als Spiralfeder ausgebildet und auf einer Führungsstange (17) angeordnet ist, welche mit ihrem ersten Ende (16) am zweiten Arm (15) des Umlenkhebels (13) gelenkig gelagert ist und mit ihrem zweiten Ende (19) in eine, im Handgriff (1) eingesetzte Stellschraube (20) hineinragt und das untere Ende (22) des Federelementes (21) auf dem Rand (23) der Stellschraube (20) anliegt.

**Claims**

1. An injection gun having a mounting (2), which is constructed for the fitting of a sleeve (8) provided for the accommodation of a cylindrical ampoule (9), and having a handle (1) connected to the said mounting (2) and an operating lever (3) attached swivelably to the handle (1) and a pressure rod (5) guided in the mounting (2) and connected to the operating lever (3) effecting the displacement of a piston (10) located in the cylindrical ampoule (9), characterized in that a pressure-limiting means acting on the operating lever (3) and the pressure rod (5) is provided with a spring element (21) and a two-arm turning lever (13), the operating lever (3) being attached to the handle (1) with interposition of the two-arm turning lever (13), the two-arm turning lever (13) being mounted swivelably on an axle (14) arranged in the handle (1), and the operating lever (3) being attached swivelably with its axis of rotation (11) on the first arm (12) of the turning lever (13) and the spring element (21) acting on the second arm (15) of the turning lever (13).

2. An injection gun according to claim 1 characterized in that the spring element (21) is constructed as a helical spring and is arranged on a guide rod (17) which, with its first end (16) is mounted in an articulated manner on the second arm (15) of the turning lever (13) and, with its second end (19), projects into an adjusting screw (20) inserted in the handle (1) and the lower end (22) of the spring element (21) bears against the edge (23) of the adjusting screw (20).

**Revendications**

1. Pistolet d'injection comportant un support (2) agencé pour permettre l'adjonction d'une douille (8) prévue pour la réception d'une ampoule cylindrique (9), une poignée (1) reliée audit support (2) et un levier de manœuvre (3) fixé de manière à pouvoir pivoter sur ladite poignée ainsi qu'une tige de compression (5) passant à l'intérieur du support (2), reliée au levier de manœuvre (3) et ayant pour effet le déplacement d'un piston (10) se trouvant à l'intérieur de l'ampoule cylindrique (9), caractérisé par le fait qu'il comprend un dispositif de limitation de la pression agissant sur le levier (3) et la tige (5) comportant un élément élastique (21) et un levier de renvoi (13) à deux branches ledit levier de renvoi (13) étant intercalé entre le levier de manœuvre (3) et la poignée (1), ledit levier (13) étant monté de manière à pouvoir pivoter sur un axe (14) disposé dans la poignée (1) et le levier de manœuvre (3) étant fixé de manière à pouvoir pivoter par son axe de rotation (11) sur le premier bras (12) du levier de renvoi (13), l'élément élastique (21) venant en prise avec le deuxième bras (15) dudit levier de renvoi (13).

2. Pistolet d'injection selon la revendication 1, caractérisé par le fait que l'élément élastique (21) est réalisé sous la forme d'un ressort en spirale et est disposé sur une tige de guidage (17) laquelle est montée de manière articulée par sa première extrémité (16) sur le deuxième bras (15) du levier de renvoi (13) et par sa deuxième extrémité (19) pénètre dans une vis de réglage (20) insérée dans la poignée (1), l'extrémité inférieure (22) de l'élément élastique (21) s'appliquant sur le bord (23) de la vis de réglage (20).